# EUROPEAN PATENT SPECIFICATION

(11) **EP 1 041 153 B1**
(45) Date of publication and mention of the grant of the patent: **24.09.2003**
(21) Application number: 98204499.2
(22) Date of filing: 14.12.1998
(51) Int. Cl.: C12P 7/08

(54) **Cheese whey treatment and valorisation process with continuous ethanolic fermentation**
Verwerten von Süssmolke duch Kontinuierliches Verfahren zur Äthanolproduktion
Traitement de petit lait et procédé de valorisation avec fermentation éthylique en continu

(30) Priority: 22.12.1997 EP 97670005
(43) Date of publication of application: 04.10.2000
(73) Proprietor: Quinta dos Ingleses, Agro-Industria, Lda., 4620 Lousada (PT)
(72) Inventor: Magalhaes Gomes Mota, Manuel Jose, Campus Gualtar 4710-057 BRAGA (PT); Couto Teixeira, Jose Antonio, Campus Gualtar 4710-057 BRAGA (PT)
(74) Representative: Pereira da Cruz, Joao

(56) References cited:
- EP-A- 0 199 634
- EP-A- 0 213 005
- GB-A- 2 021 639
- US-A- 4 346 113
- US-A- 4 748 123
- J. TEIXEIRA ET AL.: "Continuous ethanol production by a flocculating strain of Kluyveromyces marxianus: bioreactor performance" BIOPROCESS ENGINEERING, vol. 5, no. 3, 1990, pages 123-127, XP002109665
- PATENT ABSTRACTS OF JAPAN vol. 012, no. 258 (C-513), 20 July 1988 (1988-07-20) & JP 63 044880 A (HITACHI ZOSEN CORP), 25 February 1988 (1988-02-25)
- DATABASE WPI Section Ch, Week 9604 Derwent Publications Ltd., London, GB; Class D13, AN 96-038607 XP002109071 & RU 2 036 231 C (KOLODKIN A M), 27 May 1995 (1995-05-27)
- PATENT ABSTRACTS OF JAPAN vol. 016, no. 098 (C-0918), 11 March 1992 (1992-03-11) & JP 03 278897 A (T D II:KK), 10 December 1991 (1991-12-10)
- DATABASE WPI Section Ch, Week 9310 Derwent Publications Ltd., London, GB; Class B04, AN 93-079418 XP002109072 & JP 05 023149 A (MEIJI MILK PROD CO LTD), 2 February 1993 (1993-02-02)
- PATENT ABSTRACTS OF JAPAN vol. 014, no. 539 (C-0782), 28 November 1990 (1990-11-28) & JP 02 227192 A (TAX ADM AGENCY;OTHERS: 01), 10 September 1990 (1990-09-10)
- DATABASE FSTA [Online] INTERNATIONAL FOOD INFORMATION SERVICE (IFIS), FRANFURT/MAIN, DE YOUNG-SUP SHIM ET AL: "Alcohol fermentation of cheese whey by Kluyveromyces marxianus and lactic acid bacteria." Database accession no. 1999-00-p1058 XP002109668 & KOREAN JOURNAL OF FOOD SCIENCE AND TECHNOLOGY, vol. 30, no. 1, 1998, pages 161-167,
- DATABASE FSTA [Online] INTERNATIONAL FOOD INFORMATION SERVICE (IFIS), FRANFURT/MAIN, DE SUZUKI O ET AL: "Utilization of thermotolerant and flocculent yeast for wastewater treatment." Database accession no. 92-1-01-b0146 XP002109669 & HAKKOKOGAKU KAISHI, vol. 69, no. 2, 1991, pages 83-87, Toho Development Engineering Co., 3-5 Hachobori 3-chome, Chuo-ku, Tokyo 104, Japan
- DATABASE FSTA [Online] INTERNATIONAL FOOD INFORMATION SERVICE (IFIS), FRANFURT/MAIN, DE MOTA M J ET AL: "Utilization of an external loop bioreactor for the isolation of a flocculating strain of Kluyveromyces marxianus." Database accession no. 90-1-10-a0084 XP002109670 & CURRENT MICROBIOLOGY, vol. 20, no. 4, 1990, pages 209-214, Cent. de Engenharia Quimica ((INIC
- CHEMICAL ABSTRACTS, vol. 103, no. 5, 5 August 1985 (1985-08-05) Columbus, Ohio, US; abstract no. 36147, VIENNE, PATRICE ET AL: "Metabolic, physiological and kinetic aspects of the alcoholic fermentatio of whey permeate by Kluyveromyces fragilis NRRL 665 and Kluyveromyces lactis NCYC 571" XP002109666 & ENZYME MICROB. TECHNOL. (1985), 7(6), 287-94 ,1985,
- CHEMICAL ABSTRACTS, vol. 112, no. 17, 23 April 1990 (1990-04-23) Columbus, Ohio, US; abstract no. 156604, TEIXEIRA, JOSE A. ET AL: "Experimental assessment of internal diffusion limitations in yeast flocs" XP002109667 & CHEM. ENG. J. (LAUSANNE) (1990), 43(1), B13-B17 ,1990,
- PATENT ABSTRACTS OF JAPAN vol. 007, no. 242 (C-192), 27 October 1983 (1983-10-27) & JP 58 129986 A (HITACHI ZOSEN KK), 3 August 1983 (1983-08-03)
- VIENNE P.; VON STOCKAR U.: 'Alcohol from whey permeate: strain selection, temperature, and medium optimization' BIOTECHNOLOGY AND BIOENGINEERING SYMPOSIUM vol. 13, 1983, pages 421 - 435

## Description

### Field of the Invention

This invention describes a process for a cheese whey treatment and valorisation plant that incorporates a continuously operating concentric internal tube airlift bioreactor with a sedimentation zone at the top. A lactose fermenting yeast with the ability to form flocs is used. In this process a high protein concentration effluent for further processing and food grade ethanol are obtained, with a reduction in the cheese whey pollutant charge.

### Background of the Invention

Patrice Vienne *et al*. (Enzyme Microb. Technol., (1985), 7(6), 287-94, and Biotechnology and Bioengineering Symposium, 1983, vol. 13, pages 421-35) refer to methods for recycling whey, using stirred tank systems. In one of the alternatives, under operating conditions, the used strains (*Kluyveromyces fragilis* 665 and *Kluyveromyces lactis* NCYC 571), have not the ability to flocculate and the biomass retention is obtained by using an ultrafiltration device. In other alternative, where a flocculating strain is applied (only for the case of *Kluyveromyces lactis* NCYC 571) an exterior sedimentation unit and a peristaltic pump for biomass recycling are used. In both articles is referred to that the whey permeate is supplemented with yeast extract.

Kolodkin A. M., *el al*. (RU 2036231) refer to a process for obtaining ethanol from milk whey. It is a batch process, with no protein removal from cheese whey prior to fermentation and a mixed culture of two lactose fermenting yeast strains is used.

Young-Sup Shim. et al (Journal of Food Science and Technology, vol. 30, No. 1, 1998, pages 161-167) also refer to a batch process for obtaining ethanol from cheese whey. This process uses a mixed culture of a bacteria and a yeast.

Michel Henry *et al*. (EP-A-0 199 634) describe a method and device for producing ethanol by alchoolic fermentation. A non-airlift bioreactor is used in said process and complementary devices are used to retain the flocculated biomass.

### Summary of the Invention

The main objective of this invention is the implementation of a process that, when compared with existing plants, allows for the obtention of a higher productivity in the alcoholic fermentation with decreased operating costs.

The biorector used in the fermentation process is the main innovative characteristic of this invention, since all the others operations involved are similar to already existing processes.

The proposed unit for cheese whey valorisation is implemented at pilot plant scale. It includes the following processes: after being stored in a retention tank, cheese whey is fed to a 40000 MWCO organic membranes ultrafiltration unit where two fractions are obtained - the permeate and the retentate. Whenever necessary, cheese whey is pasteurised. The protein rich fraction - retentate - is stored in a tank for further processing. The deproteinized cheese whey is fed continuously to the bioreactor where fermentation occurs. The outlet flow of the biorector, with a 2.5% (w/v) ethanol content is stored in a retention tank for distillation in a batch distillation unit. The solid residues of the distillation process as well as the biomass purged from the bioreactor are treated in an existing solid wastes treatment plant.

### Brief Description of the Figure

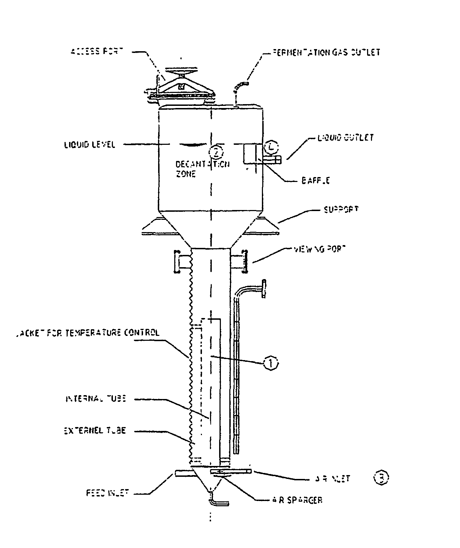

In fig. 1, the bioreactor used in the conversion of lactose from deproteinized cheese whey to ethanol is presented. Dimensions are also indicated.

### Detailed Description of the Invention

The bioreactor has a total volume of 1000 litres and is composed of two main zones: a central body formed by two concentric tubes and a decantation zone (2). The bioreactor is a concentric tube airlift bioreactor, wherein the air used to promote biomass circulation is injected at the bottom of the interior tube (3), after adequate sterilisation. Air is fed through a perforated disc (diameter - 130 mm) with 4 mm holes.

This process is a continuous fermentation process that allows for the obtention of 12 gₑₜₕₐₙₒₗl⁻¹h⁻¹ productivities

Inside and outside tube diameters are, respectively, 180 and 392 mm, in order to assure an adequate biomass circulation for high cell densities. Thus, the diameter ratio between internal and external tubes is 2.18.

The decantation zone is an enlarged decantation zone at the top of the bioreactor, forming a 60° angle with the main body of the bioreactor

In the top part (2) degassing of the liquid phase and biomass decantation occur. Biomass is recirculated to the central body of the bioreactor. In this way, biomass accumulation is improved and the biomass in the outflow is minimised. When an excessive biomass accumulation occurs, purges are done.

Biomass retention is improved by the use of a retention device - baffle - installed in the decantation zone near the effluent outlet of the bioreactor.

Temperature and pH control systems are also installed.

A lactose fermenting yeast - Kluyveromyces marxianus ATCC 10022 - with the ability to form flocs in the continuously operating bioreactor is used. This yeast property allows for the obtention of high biomass concentrations in the bioreactor and its sedimentation ability is used at the top of the bioreactor.

The conjugation of these two factors - use of a flocculating yeast strain and an innovative bioreactor design - makes possible to continuously ferment lactose to ethanol with higher productivities that the conventional batch processes.

## Claims

1. Cheese whey treatment and valorisation process, **characterised by** the use of a bioreactor composed of two main zones:
- a central body formed by two concentric tubes; and
- a decantation zone,
wherein the conversion of lactose to ethanol is done using a flocculating lactose fermenting yeast - Kluyveromyces marxianus ATCC 10022.

2. Cheese whey treatment and valorisation process according to claim 1, **characterized by** the use of a continuous fermentation process.

3. Cheese whey treatment and valorization process according to claim 1 or 2, **characterized by** the decantation zone is an enlarged decantation zone at the top, forming a 60° angle with the main body of the bioreactor.

4. Cheese whey treatment and valorisation process according to any one of claims 1 to 3, **characterised by** the bioreactor is a concentric tube airlift bioreactor, wherein the air is injected in the bottom of the internal tube being 2.18 the diameter ratio between internal and external tubes.

5. Cheese whey treatment and valorisation process according to any one of claims 1 to 4, **characterised by** sedimentation of said yeast is used at the top of the bioreactor.

6. Cheese whey treatment and valorization process according to any one of claims 1 to 5, **characterised by** the placement of a baffle in the decantation zone near the effluent outlet of the bioreactor.

## Patentansprüche

1. Verfahren zur Behandlung und Aufwertung von Käsemolke, **gekennzeichnet durch** die Verwendung eines Bioreaktors, der aus zwei Hauptzonen besteht:
- einem zentralen Körper, der aus zwei konzentrischen Röhren geformt ist; und
- einer Dekantierzone,
worin die Umwandlung von Laktose zu Äthanol unter Verwendung einer ausflockenden Laktose vergärenden Hefe - Kluyveromyces marxianus ATCC 10022 durchgeführt wird.

2. Verfahren zur Behandlung und Aufwertung von Käsemolke gemäß Anspruch 1, **gekennzeichnet durch** die Verwendung eines kontinuierlichen Fermentationsverfahrens.

3. Verfahren zur Behandlung und Aufwertung von Käsemolke gemäß Anspruch 1 oder Anspruch 2, **dadurch gekennzeichnet, dass** die Dekantierzone eine vergrößerte Dekantierzone am oberen Ende ist, die einen 60°-Winkel mit dem Hauptkörper des Bioreaktors bildet.

4. Verfahren zur Behandlung und Aufwertung von Käsemolke nach einem der Ansprüche 1 bis 3, **dadurch gekennzeichnet, dass** der Bioreaktor ein konzentrischer Röhren-Airlift-Bioreaktor ist, worin die Luft am oberen Ende der inneren Röhre eingeblasen wird, und das Verhältnis des Durchmessers von innerer und äußerer Röhre 2,18 ist.

5. Verfahren zur Behandlung und Aufwertung von Käsemolke nach einem der Ansprüche 1 bis 4, **dadurch gekennzeichnet, dass** die Sedimentation der Hefe am oberen Ende des Bioreaktors durchgeführt wird.

6. Verfahren zur Behandlung und Aufwertung von Käsemolke nach einem der Ansprüche 1 bis 5, **dadurch gekennzeichnet, dass** eine Ablenkplatte in der Dekantierzone nahe des Abfluss-Auslasses des Bioreaktors angeordnet ist.

## Revendications

1. Procédé de traitement et de mise en valeur de lactosérum de fromage, **caractérisé par** l'utilisation d'un bioréacteur composé de deux zones principales :
- un corps central formé par deux tubes concentriques ; et
- une zone de décantation,
dans lequel la conversion du lactose en éthanol est réalisée en utilisant une levure de fermentation floculante du lactose - Kluyveromyces marxianus ATCC 10022.

2. Procédé de traitement et de mise en valeur de lactosérum de fromage selon la revendication 1, **caractérisé par** l'utilisation d'un procédé de fermentation en continu.

3. Procédé de traitement et de mise en valeur de lactosérum de fromage selon la revendication 1 ou 2, **caractérisé en ce que** la zone de décantation est une zone de décantation agrandie dans sa partie supérieure, et forme un angle de 60° avec le corps principal du bioréacteur.

4. Procédé de traitement et de mise en valeur de lactosérum de fromage selon l'une quelconque des revendications 1 à 3, **caractérisé en ce que** le bioréacteur est un bioréacteur à agitation par circulation d'air à tube concentrique, dans lequel de l'air est injecté dans le fond du tube intérieur, et le rapport de diamètre entre les tubes intérieur et extérieur est de 2,18.

5. Procédé de traitement et de mise en valeur de lactosérum de fromage selon l'une quelconque des revendications 1 à 4, **caractérisé en ce que** la sédimentation de ladite levure est utilisée dans la partie supérieure du bioréacteur.

6. Procédé de traitement et de mise en valeur de lactosérum de fromage selon l'une quelconque des revendications 1 à 5, **caractérisé par** le placement d'une chicane dans la zone de décantation près de la sortie des effluents du bioréacteur.
